Europäisches Patentamt

European Patent Office

Office européen des brevets

(19)

(11) Publication number: **0 183 181**
A2

# (12) EUROPEAN PATENT APPLICATION

(21) Application number: **85114769.4**

(22) Date of filing: **21.11.85**

(51) Int. Cl.⁴: **G 01 N 33/72**
**//G01N33/50**

(30) Priority: **28.11.84 IT 2377984**

(43) Date of publication of application:
**04.06.86 Bulletin 86/23**

(84) Designated Contracting States:
**DE FR**

(71) Applicant: **CHEMICAL LABORATORIES S.r.l.**
**Via Clemente Prudenzio 14**
**I-20138 Milano(IT)**

(72) Inventor: **Torelli, Giorgio**
**Via Wolf Ferrari 5**
**I-20141 Milano(IT)**

(74) Representative: **Modiano, Guido et al,**
**MODIANO, JOSIF, PISANTY & STAUB Modiano &**
**Associati Via Meravigli, 16**
**I-20123 Milan(IT)**

(54) **Process for the analysis of haemoglobin Hba2.**

(57) The invention relates to a process for the analysis of haemoglobin HbA₂ characterized in that it comprises the following steps
a) mixing an anionic resin with a hemolysate of blood;
b) agitation of the mixture obtained in (a), to obtain separation in two phases;
c) filtration of the light phase containing haemoglobin HbA₂; and
d) photometric analysis of the filtrate obtained in (c).
The process permits the obtainment of a particularly rapid analysis of haemoglobin HbA₂ and allows the carrying out of batch analyses.

EP 0 183 181 A2

"Process for the analysis of haemoglobin HbA₂"

The present invention relates to a process for the analysis of haemoglobin $HbA_2$, which for simplicity will be referred to hereinafter as haemoglobin $A_2$.

Processes for the analysis of haemoglobin $A_2$ are known in chemical - clinical practice.

Such known processes avail themselves of electrophoresis or microchromatography on micro-columns. One may see them in particular in: EFREMOV CD; TH HUISMAN; K. BOSMAN and RN. WRIGHSTONE, Microchromatography of haemoglobins, II A rapid method for the determination of haemoglobin $A_2$, J. Lab. Clin. Med. 1974, 83, 657-664, HUISMAN T.H.J SCHROEDER W.A., BRODLE A.N., MAYSON S.M., and J. JAKWAY, Microchromatography of haemoglobins. A simplified procedure for the determination of haemoglobin $A_2$, J. Lab. Clin Med. 1975, 87, 700-712; and HUISMAN T.H.J. and DUZY AM, Studies in the heterogenity of haemoglobin. IV Microchromatographic behaviour of different human haemoglobins on anionic - exchange cellulose (DEAE Cellulose), J. Chromatogra 1962, 7, 180-203.

Both of the prior processes are laborious because they require a great deal of manual intervention and long waiting times.

It is the aim of this invention to overcome the above cited inconveniences by realizing a process for the analysis of haemoglobin $A_2$, which permits automation of the process of analysis and reduces the technical waiting times for obtainment of the results.

This aim and other objects are achieved by the process, for the analysis of haemoglobin $A_2$ according to the invention which is characterized in that it comprises the following steps:

a) mixing an anionic resin with a hemolysate of blood, in a vessel;

b) agitation of the mixture obtained in (a), to obtain separation of said mixture into a heavy phase and an overlying light phase;

c) filtration of said light phase; and

d) photometric analysis of the filtrate obtained at step (c).

Advantageously the process comprises the addition at the step (a) of an acqueous solution of glycine. That solution preferably has a concentration comprising between 0.05 and 0.3 moles/L and being stopped at a pH value comprised between 7.1 and 7.6.

The anionic resin is preferably used in a concentration of between 50-400 g/L.

The process according to the present invention will be more clearly understood from the description and the accompanying drawing figures wherein:

Figure 1 is a side view of the plunger filter according to the invention;

Figure 2 is a side view of the filter of figure 1 after the termination of the filtering process;

Figure 3 graphically illustrates the linearity of the method;

Figure 4 illustrates the correlation between the microchromatographic method and that according to the invention; and

Figure 5 illustrates a comparison between data obtained by microchromatography with that of a method product of the Isolab Company.

Table 1 illustrates the results of tests carried out on a sample of high $HbA_2$ content.

Table 2 illustrates the tests carried out on a sample of low $HbA_2$ content.

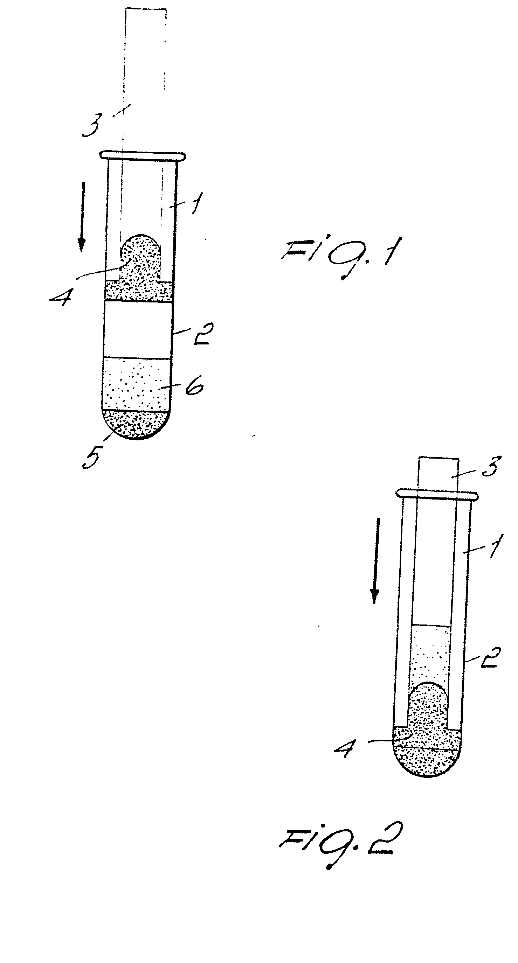

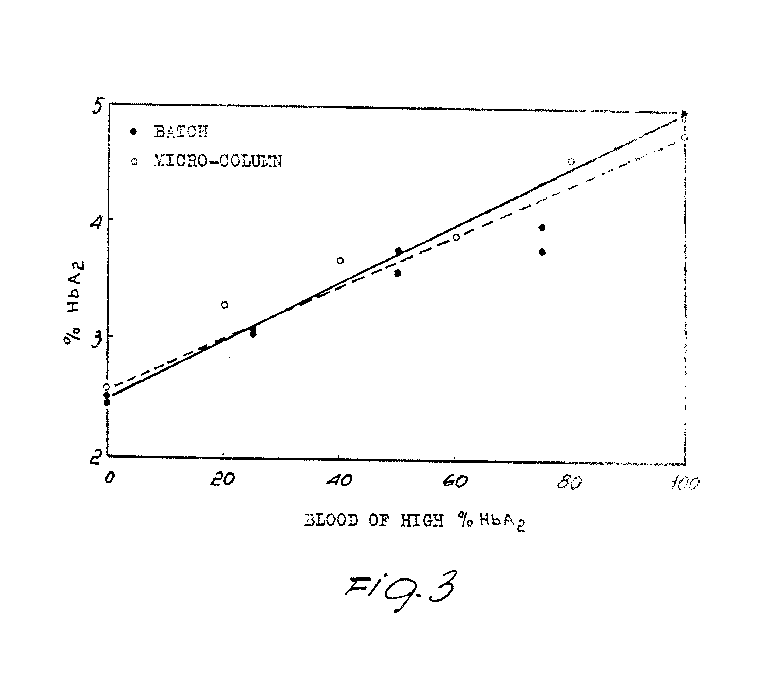

With reference to figures 1 and 2 one provides a container comprising for example a test tube 2, and a piston or plunger 1 having a cavity 3. The piston supporting at its extremity a filter 4.

Figure 1 illustrates the condition of the test tube after the agitation treatment and the first of the filtering treatments. The contents of the test tube comprise a light phase 6 overlying a heavy phase 5. The piston is adapted to be pushed, generally manually, in the direction of the arrow.

With reference to figure 2, the overlying phase 6 already presents filtrate at the interior of the cavity 3 of the plunger.

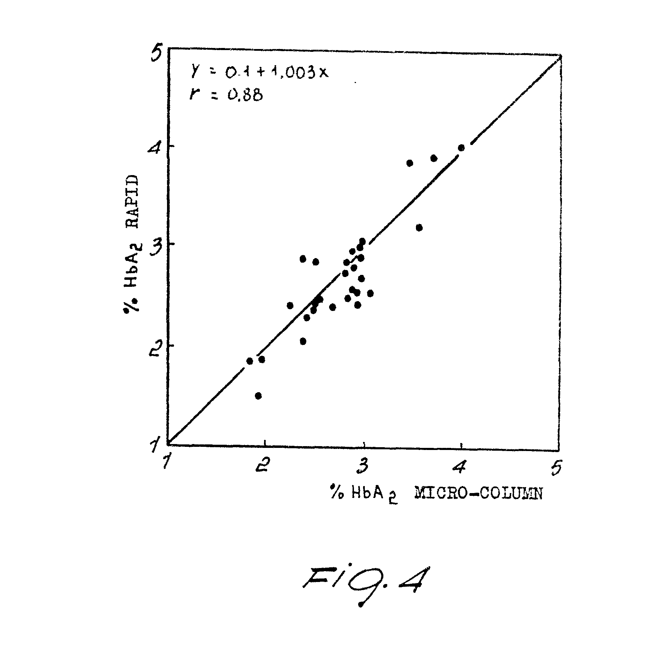

With reference to figure 3, the linearity of the method is illustrated. The linearity of the method has been determined by advantageously mixing a hemolysate of blood of low haemoglobin $A_2$ content with a hemolysate of blood having a high haemoglobin $A_2$ content.

The results obtained by the method according to the present invention are reported together with those obtained by the prior art method of microchromatography, the results as illustrated in figure 3 show that the linearity is acceptable.

With reference to figure 4, one will note a good correlation (r=0.88) between the method of microchromatography and the one according to the invention.

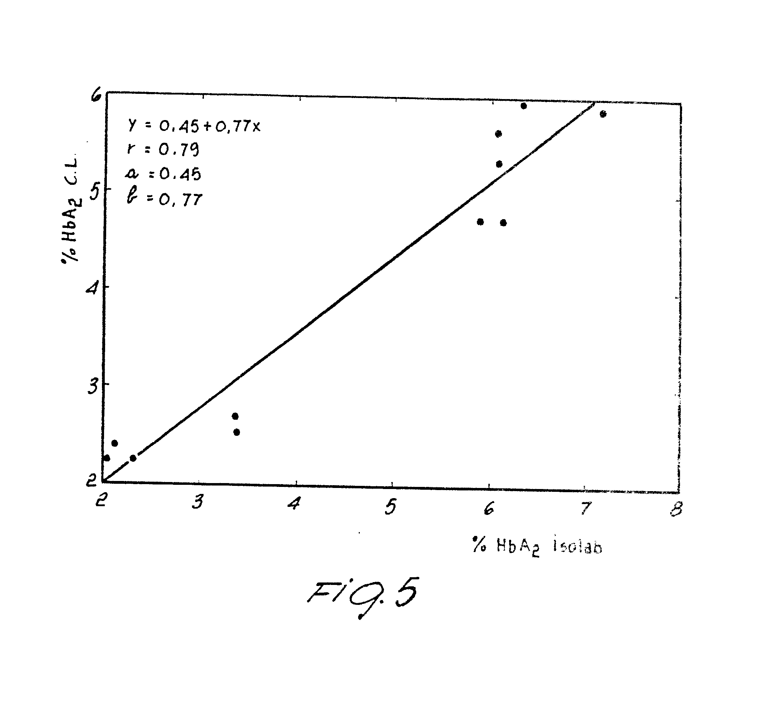

With reference to figure 5, there is illustrated data, obtained by means of the prior art method of microchromatography and related to results of an analogous method produced by the Isolab Company. The aim of this test is that of illustrating that the control method, that is the method of microchromatography according to the prior art, is comparable with that generally utilized in chemical - clinical laboratories.

The high coefficient of correlation (r=0.97) clearly indicates that the presupposed is satisfied.

With reference to table 1, illustrated is a test carried out on a sample of hemolysate of high haemoglobin $A_2$ content.

That test may be carried out by the method of microchromatography or by means of the method according to the present invention.

With reference to table 2, illustrated is a test carried out on a sample of hemolysate of low haemoglobin $A_2$ content.

That test may be carried out by the method of microchromatography or by means of the method according to the present invention.

Analysing the C-V of the tables 1 and 2 obtained, as one will note, by the batch method according to the present invention, permitting the obtainment of values acceptable in clinical practice.

Example A

100ul of hemolysate of blood are mixed with an anionic resin comprised, for example, of the resin DEAE 52. The mixture comprises an acqueous solution of glycine at a concentration of 0.1 mol/L and plugged at a pH of 7.4. The concentration of the resin is approximately 150 g/L. Anyway, favourably concentrations comprised between 50 and 450 g/L are utilized.

The mixture is lightly agitated for 5 minutes, for example by a rotating agitator. This obtains the separation of the mixture into a heavy phase and an overlying light phase containing haemoglobin $A_2$, whilst the resin has binded with the remaining haemoglobin in the heavy phase. The upper layer being separated by filtration, for example with separating filters illustrated in figures 1 and 2.

Thereafter a photometric analysis of the filtrate obtained at the step (c) is effected. The absorbance of the filtrate is preferably determined from 400nm to 425nm. This is then compared with the absorbance of the solution of hemolysate opportunely diluted.

TABLE    1

Reproducibility  of the batch method.

Sample of high HbA$_2$  concentration.
- - - - - - - - - - - - - - - - - - - -

| Number | D.O. HbA$_2$ | D.O.HbTot. | % HbA$_2$ |
|:---:|:---:|:---:|:---:|
| 1 | 0.282 | 0.800 | 4.40 |
| 2 | 0.260 | 0.800 | 4.06 |
| 3 | 0.265 | 0.800 | 4.14 |
| 4 | 0.262 | 0.800 | 4.09 |
| 5 | 0.290 | 0.800 | 4.53 |
| 6 | 0.265 | 0.800 | 4.14 |
| 7 | 0.255 | 0.800 | 3.98 |
| 8 | 0.270 | 0.800 | 4.21 |
| 9 | 0.255 | 0.800 | 3.98 |
| 10 | 0.270 | 0.800 | 4.21 |

X = 4.174              d,s, = 0,175          c,v, = 4.2%

Sample of low HbA$_2$ concentration

| Number | D.O. HbA$_2$ | D.O. HbTot. | %HbA$_2$ |
|--------|--------------|-------------|----------|
| 1 | 0.188 | 0,900 | 2.31 |
| 2 | 0.146 | 0,900 | 2,02 |
| 3 | 0.146 | 0,900 | 2,02 |
| 4 | 0.160 | 0,900 | 2,02 |
| 5 | 0,180 | 0,900 | 2,5 |
| 6 | 0,155 | 0,900 | 2,15 |
| 7 | 0,150 | 0.900 | 2,08 |
| 8 | 0.130 | 0,900 | 2,08 |
| 9 | 0.130 | 0,900 | 2,08 |
| 10 | 0,146 | 0,900 | 2,02 |

X = 2,14          d,s, = 0.174          c,v, = 8.1%

## MICRO-COLUMN REPRODUCIBILITY

Sample of low HbA$_2$ concentration.

| Number | D.O. HbA$_2$ | D.O. HbTot. | %HbA$_2$ |
|---|---|---|---|
| 1 | 0.265 | 0.555 | 2.89 |
| 2 | 0.265 | 0.550 | 2.92 |
| 3 | 0.270 | 0.560 | 2.92 |
| 4 | 0.260 | 0.530 | 2.97 |
| 5 | 0.250 | 0.540 | 2.81 |
| 6 | 0.235 | 0.510 | 2.79 |
| 7 | 0.280 | 0.570 | 2.97 |
| 8 | 0.280 | 0.570 | 2.97 |
| 9 | 0.270 | 0.550 | 2.97 |
| 10 | 0.259 | 0.530 | 2.91 |

X = 2.91          d.s. = 0.062          c.v. = 2.15%

T A B L E  2 (continued)

Sample of High $HbA_2$ concentration.

| Number | D.O. $HbA_2$ | D.O. HbTot. | %$HbA_2$ |
|---|---|---|---|
| 1 | 0.315 | 0.680 | 5.47 |
| 2 | 0.320 | 0.670 | 5.63 |
| 3 | 0.325 | 0.700 | 5.48 |
| 4 | 0.318 | 0.690 | 5.44 |
| 5 | 0.310 | 0.700 | 5.24 |
| 6 | 0.320 | 0.710 | 5.35 |
| 7 | 0.325 | 0.710 | 5.41 |
| 8 | 0.315 | 0.680 | 5.32 |
| 9 | 0.325 | 0.700 | 5.48 |
| 10 | 0.315 | 0.690 | 5.39 |

X = 5.419    d.s. = 0.1028    c.v. = 1.2%

## CLAIMS

1. A process for the analysis of haemoglobin $A_2$ ($HbA_2$) characterized in that it comprises the following steps:

a) mixing an anionic resin with a hemolysate of blood;

b) agitation of the mixture obtained in (a) to obtain separation of said mixture into a heavy phase and an overlying light phase;

c) filtration of said light phase; and

d) photometric analysis of the filtrate obtained at step (c).

2. A process according to claim 1 characterized in that said step (a) comprises the addition of an acqueous solution of glycine.

3. A process according to claim 2 characterized in that said acqueous solution of glycine has a concentration comprised between 0.05 and 0.3 mol/L.

4. A process according to claim 2 characterized in that said acqueous solution of glycine is stopped at a pH comprised between 7.1 and 7.6.

5. A process according to claim 2 characterized in that said anionic resin is present at a concentration comprised between 50 and 400 g/L.

6. A process according to claim 1, characterized in that said agitation is effected with a rotating agitator.

7. A process according to claim 1 characterized in that said filtration is carried out at the interior of said mixing vessel by means of a plunger filter.

8. A process according to claim 7 characterized in that said plunger has a cavity adapted for collecting the filtered product.

9. A process according to claim 1 characterized in that the absorbance of the filtrate is determined from 400nm to 425nm.

Fig.1

Fig.2

Fig. 3

$Y = 0.1 + 1.003x$
$r = 0.88$

% HbA₂ RAPID

% HbA₂ MICRO-COLUMN

Fig. 4

FIG. 5